# EUROPÄISCHE PATENTSCHRIFT

(11) **EP 1 086 669 B1**
(45) Veröffentlichungstag und Bekanntmachung des Hinweises auf die Patenterteilung: **08.12.2010**
(21) Anmeldenummer: 00118371.4
(22) Anmeldetag: 24.08.2000
(51) Int. Cl.: A61F 5/00, A61F 5/34

(54) **Polster mit weichelastischem Bereich und Verfahren zu dessen Herstellung**
Cushion having a flexible region and method for making same
Coussin comprenant une partie souple et son procédé de fabrication

(30) Priorität: 25.09.1999 DE 19946058
(43) Veröffentlichungstag der Anmeldung: 28.03.2001
(73) Patentinhaber: Thuasne Deutschland GmbH, 30938 Burgwedel (DE)
(72) Erfinder: Littmann, Alexander, D-30916 Isernhagen (DE)
(74) Vertreter: Siekmann, Gunnar

(56) Entgegenhaltungen:
- DE-U- 8 700 681
- FR-A- 2 712 487
- US-A- 4 700 698
- US-A- 5 027 801
- US-A- 5 306 229

## Beschreibung

Die Erfindung betrifft ein Verfahren zur Herstellung eines Polsters mit mindestens einem weichelastischen Bereich gemäß dem Oberbegriff des Patentanspruchs 1, insbesondere von Filzpolstern mit Silikonpelotten, zur Verwendung mit orthopädischen Hilfsmitteln, mit dem ein fließfähiges Material zur Bildung des weichelastischen Bereichs mit dem Polster in Kontakt gebracht wird und das fließfähige Material in dem Bereich verfestigt wird. Weiterhin betrifft die Erfindung ein solchermaßen hergestelltes Polster gemäß dem Oberbegriff des Patentanspruchs 9.

Es ist bekannt, daß derartige Polster in orthopädischen Hilfsmitteln, beispielsweise in Epicondylitisspangen eingesetzt werden. Dazu wird ein an die Form der Epicondylitisspange angepaßtes Filzpolster verwendet, das in seinen Endbereichen Silikonpunkte aufweist, die sich in besonderer Weise an die Haut des Benutzers des orthopädischen Hilfsmittels anlegen und ein Verrutschen des Polsters verhindern. Die Herstellung dieser bekannten Filzpolster erfolgt so, daß in das Filzpolster eine Ausnehmung eingebracht wird und ein vorgefertigter Silikonpunkt gleicher Größe in diese Ausnehmung eingeklebt wird.

Ein Polster der eingangs genannten Art ist aus der DE 87 00 681 U bekannt. Diese Druckschrift offenbart eine orthopädische Einlage, die ein Polster aufweist. Dieses Polster aus Silikonkautschuk kann in Vertiefungen oder Mulden des Polsters in situ gegossen werden. Dabei stellt das Polster selbst eine Form zur Aufnahme des Silikonkautschuks bereit. Alternativ wird beschrieben, daß die Polster aus Silikonkautschuk in die Vertiefungen oder Mulden des Polsters eingeklebt werden können.

Aus der US 5,027,801 ist ein Verfahren zum Erzeugen von Silikonpolstern bekannt. Auf einer Unterlage sind Basisrandstücke vorgesehen, die dort fest montiert sind und auf die vorderseitige Stücke aufgelegt sind. Über diese Stücke hinweg und in die dazwischen liegende Mulde wird ein dünner Film gelegt, der durch ein angelegtes Vakuum fest an den Untergrund angesaugt wird. In die so geschaffene Form, die mit einer Folie ausgelegt ist, wird eine Gelschicht eingefüllt. Auf diese Gelschicht wird eine rückseitige Fläche aufgebracht, die mit Hilfe eines Klebmaterials auf der dünnen Schicht bzw. der Gelschicht verklebt wird. Die hier verwendete Rückseite bildet die Basis einer Einlage.

Aus der US 4,700,698 ist eine Knieorthese bekannt, bei der innerhalb einer nicht kompressiblen Auflage Verstärkungen zur Erhöhung des Drucks in die Auflage vulkanisiert sein können. Diese Verstärkungen sind als flache Teile mit Ankerlöchern ausgebildet, durch die Teile der Auflage geführt und so befestigt sind.

Der Erfindung liegt die Aufgabe zugrunde, ein Verfahren der eingangs genannten Art zu schaffen, mit dem die Herstellung von Polstern zur Verwendung mit orthopädischen Hilfsmitteln besonders einfach und kostengünstig ist und besonders zuverlässig am Körper des Benutzers positionierbar ist.

Diese Aufgabe wird verfahrensmäßig mit den Merkmalen des Patentanspruchs 1 gelöst. Die Merkmale des erfindungsgemäßen Polsters sind im Patentanspruch 9 angegeben. Vorteilhafte Weiterbildungen der Erfindung sind in den Unteransprüchen beschrieben.

Nach dem Grundgedanken der Erfindung wird ein fließfähiges Material zur Bildung des weichelastischen Bereichs mit dem Polster in Kontakt gebracht und dieses fließfähige Material wird in dem Bereich verfestigt. Dadurch kann auf äußerst einfache Weise schnell und preiswert ein Polster mit einem weichelastischen Bereich hergestellt werden.

Erfindungsgemäß wird das Polster mit einer Ausnehmung auf eine Auflagefläche aufgelegt und anschließend wird in die Ausnehmung ein fließfähiges Material eingefüllt, das die weichelastische Einlage bildet. Das fließfähige Material verläuft in der Ausnehmung des Polsters und wird dann verfestigt. Das Polster wird dabei zwischen der Auflagefläche und einem Polsterhalter angeordnet, wobei der Polsterhalter eine zu dem vorgesehenen weichelastischen Bereich korrespondierende Ausnehmung aufweist, und das fließfähige Material wird in den auf den Polster liegenden Polsterhalter bis zu einer Höhe eingefüllt, die über die Höhe des Polsters hinausgeht. Auf diese Weise wird besonders einfach und schnell ein Polster mit einer weichelastischen Einlage geschaffen, bei dem das Polster und die weichelastische Einlage miteinander verbunden sind. Dies geschieht durch das Verlaufen des fließfähigen Materials innerhalb der Ausnehmung, wobei das fließfähige Material bis zu dem Polster verläuft und dort anhaftet und verfestigt. Auf diese Weise wird in einem Arbeitsgang eine größenmäßig genau passende Einlage hergestellt, und gleichzeitig auf eine ideale Weise mit dem Polster verbunden.

In einer besonders bevorzugten Ausführungsform des erfindungsgemäßen Verfahrens wird die weichelastische Einlage aus Silikon hergestellt, das in flüssiger Form in die Ausnehmung eingefüllt und dann ausvulkanisiert wird.

Weiterhin ist es günstig, wenn das in die Ausnehmung eingefüllte fließfähige Material aktiv verteilt wird. Grundsätzlich ist es auch möglich, dieses alleine verlaufen zu lassen. Dabei bildet sich nach einiger Zeit ebenfalls eine gleichmäßige Schicht aus. Es ist natürlich von Vorteil, wenn die Auflagefläche absolut waagerecht ausgerichtet ist. In einer anderen Weiterbildung der Erfindung wird die Auflagefläche geheizt. Bevorzugterweise wird die Auflagefläche auf etwa 70 - 120° Celsius geheizt, so daß durch die Temperatureinstellung auf die Verfestigung des Materials bzw. die Vulkanisierung des Silikons Einfluß genommen werden kann. Die Vulkanisationszeit von Silikon beträgt je nach Temperatur und Katalysatorgehalt zwischen einer Minute und mehreren Stunden. Besonders bevorzugt ist es, die Temperatur so einzustellen, daß die Vulkanisationszeit etwa zwei bis drei Minuten beträgt, da sonst das Silikon zu stark in das Polster einzieht. Bei einer Vulkanisationszeit von zwei bis drei Minuten verläuft das Silikon bis zum Rand der Ausnehmung und zum Rand des Polsters und verwächst an den Kanten mit dem Polster und vulkanisiert aus. Nach der gewünschten Vulkanisationszeit wird das Polster von der geheizten Auflagefläche entnommen und günstigerweise gekühlt.

Erfindungsgemäß wird das Polster auf einen Polsterhalter, der eine zu der Ausnehmung im Polster korrespondierende Ausnehmung aufweist, aufgespannt und zusammen mit dem Polsterhalter auf die Auflagefläche gelegt. Dies erfolgt so, daß das Polster zwischen der Auflagefläche und dem Polsterhalter angeordnet wird, das heißt, das das Polster direkt auf der Auflagefläche liegt und der Polsterhalter obenauf angeordnet wird. Günstigerweise wird der Polsterhalter mechanisch fixiert, so daß dieser eine Kraft auf das Polster ausübt, so daß das Polster abschließend und abdichtend auf der Auflagefläche liegt und das in die Ausnehmung des Polsters eingefüllte fließfähige Material innerhalb der Ausnehmung verbleibt und nicht durch Spalten entweichen kann. Günstigerweise wird dazu ein Polsterhalter verwendet, der in seiner Form dem Polster angepaßt ist. Dadurch wird nicht nur die Ausnehmung freigegeben, sondern auch sichergestellt, daß das Polster im gesamten Bereich auf die Auflagefläche aufgedrückt werden kann.

In einer anderen bevorzugten Weiterentwicklung der Erfindung wird vor Durchführung des eingangs genannten Verfahrens auf eine Seite des Polsters ein Flausch aufgebracht. Dieser Flausch ist derart beschaffen, daß es zusammen mit einem Hakenband als Klettverschluß dient. Auf diese Weise läßt sich das Polster mit dem Flausch an dem Polsterhalter befestigen, wobei an dem Polsterhalter entsprechende Hakenbänder angeordnet sind. Das Polster wird dann mit der Seite, die der Seite mit dem Flausch gegenüberliegt, auf die Auflagefläche gelegt, wobei dann in der auf der Auflagefläche untenliegenden Polsterseite bündig abschließende Silikonpelotten oder Silikonpunkte hergestellt werden, die mit dieser Seite auch am Körper des Patienten anliegen und einem Rutschen des Polsters entgegenwirken. Erfindungsgemäß wird die Ausnehmung bis in die korrespondierende Ausnehmung des Polsterhalters hinein mit dem fließfähigen Material zur Bildung der Einlage gefüllt, so daß die Einlage auch dicker ausgebildet werden kann als das Polster selbst bzw. so daß eine Einlage mit einer beliebigen Höhe ausgebildet werden kann. Bevorzugt hat die Einlage bzw. die Silikonpelotte eine Höhe von ein bis zwei mm.

Das Verfahren wird bevorzugt so durchgeführt, daß mehrere Polsterhalter parallel betrieben werden, so daß gleichzeitig mehrere Polster auf die Auflagefläche aufgelegt werden und in die Ausnehmungen dieser Polster das fließfähige Material eingefüllt und dieses gleichzeitig verfestigt wird. Auf diese Weise ist eine Produktion großer Stückzahlen äußerst wirtschaftlich möglich.

In einer anderen bevorzugten Weiterbildung der Erfindung ist die Auflagefläche so profiliert, daß die darauf hergestellten Silikonbereiche eine gewünschte Formgebung aufweisen. Hier sind insbesondere konkave oder konvexe Gestaltungen der geheizten Auflagefläche möglich. Besonders bevorzugt ist eine konkave, also nach innen gewölbte oder hohle Gestaltung der Auflagefläche, so daß der Silikonbereich nach außen konvex geformt ist. Insgesamt wird also so ein Filzpolster mit einem nach außen gewölbten Silikonbereich hergestellt, der etwas vorsteht und daher besonders gut die Haut des Benutzers kontaktieren kann und so ein Verrutschen verhindert.

Mit dem beschriebenen Verfahren kann ein Polster hergestellt werden, das einen weichelastischen Bereich, insbesondere eine Silikonpelotte aufweist, wobei das Polster mit dem weichelastischen Bereich in einem Grenzbereich verwachsen ist. Dieser Grenzbereich ist bevorzugt etwa 1 mm stark. Der weichelastische Bereich ist an seinem Außenrand mit dem Polster verwachsen. Ein derartiges Polster ist besonders einfach, nämlich in einem Arbeitsgang, herstellbar und vermeidet ein getrenntes Verkleben des weichelastischen Bereichs mit dem übrigen Polster.

Nachfolgend wird die Erfindung anhand eines in der Zeichnung dargestellten bevorzugten Ausführungsbeispiels weiter erläutert. Im einzelnen zeigen die schematischen Darstellungen in:
Fig. 1 eine Draufsicht auf ein erfindungsgemäß hergestelltes Polster und
Fig. 2 eine Seitenansicht eines erfindungsgemäß hergestellten Polsters.

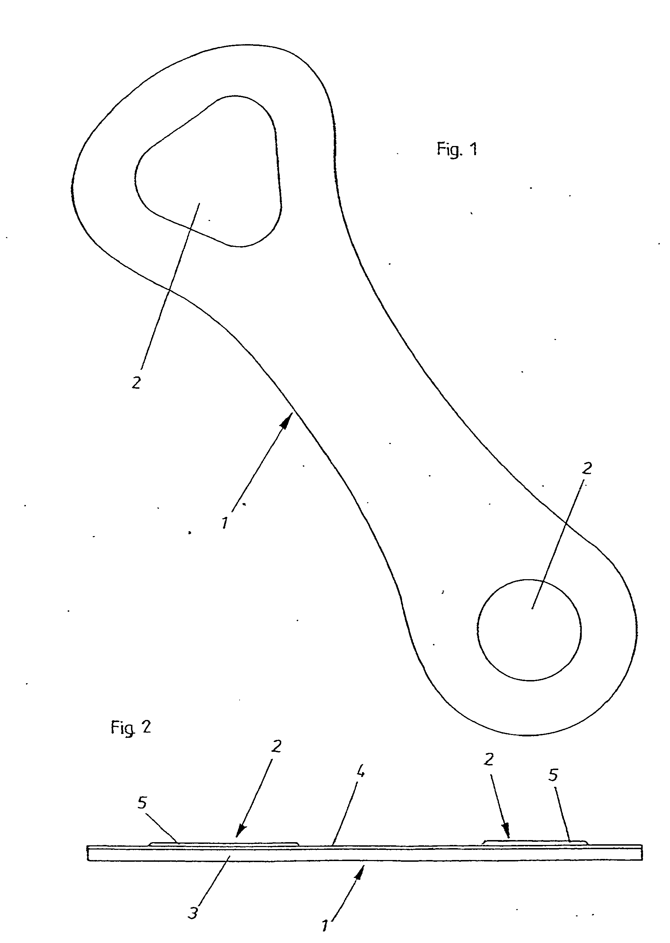

In Fig. 1 ist ein Polster 1 dargestellt, das zur Verwendung in einer Epicondylitisspange bestimmt ist. Das Polster ist in seiner Form der Form der Epicondylitisspange angepaßt und in den erweiterten Endbereichen sind weichelastische Einlagen 2 angeordnet, die auf der Haut liegen und ein Verrutschen des Polsters 1 verhindern. In Fig. 2 ist das erfindungsgemäß hergestellte Polster 1 in einer Seitenansicht dargestellt. Das Polster 1 besteht in seinem unteren Bereich aus einer Filzschicht 3, auf der ein Flausch 4 angeordnet ist, der so ausgebildet ist, daß er mit einem Hakenband verklettbar ist. Die Einlagen 2 können durch das erfindungsgemäße Verfahren so ausgebildet sein, daß sie einen gewissen Überstand 5 aufweisen, mit dem sie über die Einlage 1 hochstehen. Dieser Überstand ist in aller Regel auf der Seite, auf der der Flausch 4 angeordnet ist, da das Polster 1 während der Herstellung mit der Seite, auf der der Filz 3 angeordnet ist, auf die Auflagefläche gelegt wird.

Bei dem erfindungsgemäßen Herstellungsverfahren werden zunächst aus dem Einlagenmaterial, also bevorzugt aus einem Filzpolster mit einer darauf angeordneten Flauschschicht, Polster in der gewünschten Form und Größe ausgestanzt. Dabei werden auch Ausnehmungen für die späteren Einlagen bzw. Pelotten ausgestanzt. Das so ausgestanzte Filzpolster 1 wird mit seiner Flauschschicht 4 auf einen Polsterhalter aufgeklettet. Üblicherweise werden dabei eine Vielzahl von nebeneinander angeordneten Polsterhaltern verwendet, auf die jeweils ein Polster aufgeklettet wird. Die Polsterhalter haben dabei annähernd die gleiche Kontur wie die Polster 1. Die Polsterhalter werden dann gemeinsam mit einem Haltebügel heruntergeklappt und auf einer beheizbaren Auflagefläche festgespannt. Die Auflagefläche ist bevorzugt von einer Aluminiumplatte gebildet, die auf eine Temperatur von 70 bis 120° Celsius geheizt ist. In die Ausnehmungen des Polsters und des darüberliegenden Polsterhalters wird additionsvernetzendes Silikon mit einer entsprechenden Härteeinstellung eingefüllt. Das Silikon verläuft bis zur Filz-und Polsterhalterbegrenzung, verwächst an den Kanten mit dem Filz und vulkanisiert aus. In Abhängigkeit von der Temperatur und dem Katalysatorgehalt beträgt die Vulkanisationszeit zwischen ein und zehn Minuten bis zu mehreren Stunden. Als besonders günstig hat sich eine Vulkanisationszeit von etwa zwei bis drei Minuten herausgestellt, da dann das Silikon geradeso so weit in das Filz eingezogen ist, um mit ihm zu verwachsen. Bei einer längeren Vulkanisationszeit besteht die Gefahr, daß das Silikon zu stark in das Material des Polsters einzieht. Durch die Anordnung des Polsters 1 mit seiner Filzseite 3 auf der Auflagefläche ist sichergestellt, daß das Polster 1 mit seinen weichelastischen Einlagen, bzw. seinen Silikonpelotten, flächig und bündig ausgebildet ist. Auf der gegenüberliegenden Seite kann durch den auf dem Polster liegenden Polsterhalter Silikon oder ein anderes weichelastisches Material bis zu einer Höhe eingefüllt werden, die über die Höhe des Polsters 1 hinausgeht. Es ist also möglich, die Höhe der Silikonpelotte auf der Rückseite zu variieren. Besonders günstig ist eine Höhe von ein bis zwei mm.

## Patentansprüche

1. Verfahren zur Herstellung eines Polsters mit mindestens einem weichelastischen Bereich, insbesondere von Filzpolstern mit Silikonpelotten, zur Verwendung mit orthopädischen Hilfsmitteln, bei dem ein fließfähiges Material zur Bildung mindestens einer weichelastischen Einlage in eine Ausnehmung des Polsters eingebracht wird und das fließfähige Material in der Ausnehmung verfestigt wird,
**dadurch gekennzeichnet,**
**dass** das Polster so mit der Ausnehmung auf eine Auflagefläche gelegt wird, dass auf der der Auflagefläche zugewandten Seite das Polster mit seinen weichelastischen Einlagen flächig und bündig ausgebildet wird.

2. Verfahren nach Anspruch 1, **dadurch gekennzeichnet, dass** die Polster auf Polsterhalter, die eine zu dem vorgesehenen weichelastischen Bereich korrespondierende Ausnehmung aufweisen, aufgespannt werden.

3. Verfahren nach Anspruch 2, **dadurch gekennzeichnet, dass** das Polster zwischen Auflagefläche und Polsterhalter angeordnet wird und dass der Polsterhalter mechanisch fixiert wird.

4. Verfahren nach einem der vorhergehenden Ansprüche, **dadurch gekennzeichnet, dass** auf einer Seite des Polsters ein Flausch aufgebracht wird.

5. Verfahren nach Anspruch 4, **dadurch gekennzeichnet, dass** das Polster mit der Seite, die der Seite mit dem Flausch gegenüberliegt, auf die Auflagefläche gelegt wird.

6. Verfahren nach einem der vorhergehenden Ansprüche, **dadurch gekennzeichnet, dass** die Ausnehmung oder der weichelastische Bereich bis in die korrespondierende Ausnehmung des Polsterhalters hinein mit Silikon gefüllt wird.

7. Verfahren nach einem der vorhergehenden Ansprüche, **dadurch gekennzeichnet, dass** in die Ausnehmungen mehrerer Polsterhalter das fließfähige Material eingefüllt wird und dass das Verfestigen des fließfähigen Materials in den Ausnehmungen der verschiedenen Polsterhalter gleichzeitig durchgeführt wird.

8. Verfahren nach einem der vorhergehenden Ansprüche, **dadurch gekennzeichnet, dass** eine Auflagefläche verwendet wird, die im Bereich zur Bildung der weichelastischen Einlage eine konkave Oberflächengestaltung hat.

9. Polster mit weichelastischem Bereich, insbesondere Filzpolster mit Silikonpelotte, hergestellt nach einem Verfahren gemäß einem der Patentansprüche 1 bis 8.

10. Polster nach Anspruch 9, **dadurch gekennzeichnet, dass** der weichelastische Bereich an seinen Außenrändern in einem Grenzbereich mit dem Filzpolster verwachsen ist.

11. Polster nach Anspruch 10, **dadurch gekennzeichnet, dass** der Grenzbereich etwa 1 mm stark ist.

12. Polster nach einem der Ansprüche 9 bis 11, **dadurch gekennzeichnet, dass** der weichelastische Bereich auf einer Seite gegenüber dem Polster einen Überstand (5) aufweist.

## Claims

1. A method for manufacturing a cushion having at least one flexible region, in particular of felt cushions with silicone pads, for use with orthopaedic aids in which a flowable material in inserted into a recess of a cushion to form at least one flexible insert and the flowable material is hardened in the recess, **characterised in that** the cushion is laid with the recess on a bearing surface such that on the side facing the bearing surface the cushion is configured to be flat and flush with its flexible inserts.

2. The method according to claim 1, **characterised in that** the cushions are stretched onto cushion holders which have a recess corresponding to the provided flexible region.

3. The method according to claim 2, **characterised in that** the cushion is arranged between bearing surface and cushion holder and that the cushion holder is fixed mechanically.

4. The method according to any one of the preceding claims, **characterised in that** a fleece is attached on one side of the cushion.

5. The method according to claim 4, **characterised in that** the cushion is placed with the side opposite the side with the flange on the bearing surface.

6. The method according to any one of the preceding claims, **characterised in that** the recess or the flexible region is filled with silicone right into the corresponding recess of the cushion holder.

7. The method according to any one of the preceding claims, **characterised in that** the flowable material is poured into the recesses of a plurality of cushion holders and that the hardening of the flowable material in the recesses of the various cushion holders is carried out at the same time.

8. The method according to any one of the preceding claims, **characterised in that** a bearing surface is used which has a concave surface shape in the region to form the flexible insert.

9. A cushion having a flexible insert, in particular felt cushion having a silicone pad, manufactured according to a method according to any one of patent claims 1 to 8.

10. The cushion according to claim 9, **characterised in that** at its outer edges the flexible region is fused with the felt cushion in its boundary region.

11. The cushion according to claim 10, **characterised in that** the boundary region is about 1 mm thick.

12. The cushion according to any one of claims 9 to 11, **characterised in that** the flexible region has a protrusion (5) with respect to the cushion on one side.

## Revendications

1. Procédé de fabrication d'un coussin comportant au moins une partie souple, notamment de coussins en feutres à pelotes de silicone destinés à être utilisés avec des accessoires orthopédiques, dans lequel un matériau fluide est introduit dans un évidement du coussin pour former au moins une garniture souple et le matériau fluide est solidifié dans l'évidement,
**caractérisé en ce que**
le coussin est posé par son évidement sur une surface d'appui, de manière à ce que, sur sa face tournée vers la surface d'appui, le coussin ait une conformation surfacique et à fleur avec ses garnitures souples.

2. Procédé selon la revendication 1, **caractérisé en ce que** les coussins sont tendus sur des supports de coussins qui présentent un évidement correspondant à la partie souple prévue.

3. Procédé selon la revendication 2, **caractérisé en ce que** le coussin est disposé entre la surface d'appui et le support de coussin et que le support de coussin est fixé mécaniquement.

4. Procédé selon une des revendications précédentes, **caractérisé en ce qu'**un tissu pelucheux est appliqué sur un côté du coussin.

5. Procédé selon la revendication 4, **caractérisé en ce que** le coussin est posé sur la surface d'appui par le côté opposé au côté recouvert du tissu pelucheux.

6. Procédé selon une des revendications précédentes, **caractérisé en ce que** l'évidement ou la partie souple est rempli de silicone jusque dans l'évidement correspondant du support de coussin.

7. Procédé selon une des revendications précédentes, **caractérisé en ce que** le matériau fluide est introduit dans les évidements de plusieurs supports de coussins et que la solidification du matériau fluide dans les évidements des différents supports de coussins est réalisée en même temps.

8. Procédé selon une des revendications précédentes, **caractérisé en ce qu'**on utilise une surface d'appui qui a une conformation surfacique concave au niveau de formation de la garniture souple.

9. Coussin à partie souple, notamment coussin en feutre à pelote de silicone, fabriqué suivant un procédé selon une des revendications 1 à 8 du brevet.

10. Procédé selon la revendication 9, **caractérisé en ce que** la partie souple, au niveau de ses bords extérieurs, se confond au coussin de feutre dans une zone limite.

11. Procédé selon la revendication 10, **caractérisé en ce que** la zone limite a environ 1 mm d'épaisseur.

12. Procédé selon une des revendications 9 à 11, **caractérisé en ce que** la partie souple présente un rehaussement (5) sur une face opposée au coussin.
